# EUROPÄISCHE PATENTSCHRIFT

(11) **EP 0 913 165 B1**
(45) Veröffentlichungstag und Bekanntmachung des Hinweises auf die Patenterteilung: **28.02.2007**
(21) Anmeldenummer: 98108526.9
(22) Anmeldetag: 11.05.1998
(51) Int. Cl.: A61M 25/01

(54) **Stülpschlauchkonstruktion**
Eversion catheter construction
Construction de cathéter d'éversion

(30) Priorität: 03.11.1997 DE 19748500
(43) Veröffentlichungstag der Anmeldung: 06.05.1999
(73) Patentinhaber: invendo medical GmbH, 69469 Weinheim (DE)
(72) Erfinder: Viebach, Thomas, 82282 Pischertshofen (DE); Pauker, Fritz, 86316 Wiffertshausen/Friedberg (DE); Weiglhofer, Gerhard, 86947 Schwabhausen/Weil (DE); Pauker, Robert, 86438 Kissing (DE)
(74) Vertreter: TBK-Patent

(56) Entgegenhaltungen:
- WO-A-88/01924
- AT-B- 366 904
- US-A- 5 171 305
- US-A- 5 676 688

## Beschreibung

Die vorliegende Erfindung bezieht sich auf eine Stülpschlauchkonstruktion vorzugsweise für ein Endoskopiegerät, einen Katheter oder ein anderem schaftähnliches Rundmaterial zur Untersuchung von kanalartigen Hohlräumen z. B. am menschlichen Körper oder für das Einführen von OP-Werkzeugen Medikamenten usw. und insbesondere eine Stülpschlauchkonstruktion gemäss dem Oberbegriff des Patentanspruchs 1.

Gemäß einem Haus internen Stand der Technik hat die Erfinderin ein Endoskopiegerät in Entwicklung, das ein Doppelstopschlauchsystem gemäß vorstehender Gattung verwendet, wie es nachfolgend kurz beschrieben wird:

Dieses Endoskopiegerät hat einen Endoskopschaft, der in einem beidseitig gestülpten Schlauch gleitend geführt ist, der wiederum durch eine Antriebseinrichtung fortbewegbar ist, die auf den inneren Schlauchabschnitt des Stülpschlauchs einwirkt. Die Antriebseinrichtung hat zumindest ein kontinuierliches Vorschubmittel, insbesondere Reibräder, die radial auf den inneren Schlauchabschnitt pressbar sind, um diesen in Axialrichtung des Schafts im wesentlichen kontinuierlich zu bewegen. Dies hat den großen Vorteil, dass der kontinuierliche Vortrieb des Stülpschlauchsystems exakt steuerbar und damit beispielsweise das distale Ende des Endoskops ortsgenau führbar ist.

Hierbei ist es vorgesehen, dass die Anpresskraft des Vorschubmittels auf den inneren Schlauchabschnitt so gewählt ist, dass der Schaft zumindest im Bereich des Vorschubmittels im direkten Reibkontakt mit dem inneren Schlauchabschnitt ist. Das Vorschubmittel wird von einem oder mehreren Reibrädern gebildet, die gegen den inneren Schlauchabschnitt mit einer vorbestimmten oder einstellbaren Anpresskraft vorgespannt sind, so dass zum einen ein kontinuierlicher und zum anderen ein möglichst schlupffreier Vorschub des Endoskopschafts in den zu untersuchenden Hohlraum eines Patienten gewährleistet ist.

Des weiteren hat die Antriebseinrichtung eine Vorrichtung zur Synchronisation der Schaftbewegung mit der Stülpschlauchbewegung. Diese kann ein am Schaft axial fixiertes, hinteres und vorderes End- oder Klemmstück sein, an dem gleitfähig je nach Vorschubrichtung der hintere oder vordere Stülpbereich des Stülpschlauchs fest anliegt, so dass der Stülpschlauch über das hintere oder vordere Endstück eine Bremskraft entgegen der gerade vorherrschenden Vorschubkraft des Vorschubmittels auf den Endoskopschaft aufbringt.

Zwischenzeitlich durchgeführte Versuche der Erfinderin haben gezeigt, dass bei einem derart aufgebauten Endoskopiegerät mit dem vorstehend beschriebenen Stülpschlauchsystem die über die Reibräder aufbringbaren Vorschubkräfte jedoch begrenzt sind. Der Grund hierfür besteht darin, dass zum einen die Vorschubkräfte der Reibräder über den inneren Schlauchabschnitt nur teilweise auf den Endoskopschaft aufgebracht werden können, da sich zwischen dem inneren Schlauchabschnitt und der Mantelfläche des Endoskopschafts ein Schmierfilm aufbaut, der eine relative Gleitbewegung zwischen dem Endoskopschaft und dem inneren Schlauchabschnitt ermöglicht. D. h., dass ein anderer Teil der Vorschubkraft der Reibräder über den inneren Schlauchabschnitt auf das vordere Endstück einwirkt, welches wiederum an dem Endoskopschaft angeklemmt ist. Zum anderen wirken die gesamten Bremskräfte, welche an dem hinteren End- oder Klemmstück des Endoskopschafts entgegen der Vorschubkraft der Reibräder entstehen, auf den inneren und äußeren, hinteren Schlauchabschnitt des Stülpschlauchs ein.

Der vorstehend beschriebene Stülpschlauch besteht im wesentlichen aus einem Silikon oder einem ähnlichen Material, und weist eine solche Dicke auf, die ein möglichst verlustfreies Umstülpen an den vorderen und hinteren Umstülpbereichen während einer Bewegung des Endoskopschafts zulässt. Diese Konstruktion erlaubt jedoch nur eine verhältnismäßig geringe Schub- oder Scherbelastung insbesondere des inneren Schlauchabschnitts in Vorschubrichtung des Endoskopschafts durch die Reibräder bzw. durch das hintere und vordere Klemmstück, wobei bei einer Überschreitung einer vom Material und dessen Dicke abhängigen, maximal zulässigen Belastungsgrenze ein Aufschuppen insbesondere des inneren Schlauchabschnitts auftritt. In diesem Zustand wird nicht nur die Relativgleitfähigkeit verschlechtert, sondern es verringert sich auch die auf den Endoskopschaft maximal aufbringbare Vorschubkraft, so dass sich die Vorschubbewegung verlangsamt oder sogar stoppt.

Im Ergebnis dieser Untersuchungen ist festzustellen, dass bei einer Stülpschlauchkonstruktion gemäss vorstehender Beschreibung die Eindringtiefe des Endoskops in den zu untersuchenden Hohlraum begrenzt ist, da mit zunehmender Eindringtiefe eine sich erhöhende Vorschubkraft auf den Endoskopschaft aufgebracht werden muss, welche wiederum durch die maximale Belastungsfähigkeit insbesondere des inneren Schlauchabschnitts des Stülpschlauchs begrenzt ist.

Im Oberbegriff des Patentanspruchs 1 wird indessen von einer Stülpschlauchkonstruktion ausgegangen, wie sie aus der WO 88/01924 bekannt ist.

Die dort gezeigte Stülpschlauchkonstruktion besteht aus einem inneren Schlauchabschnitt, der an vorderen und hinteren Umstülpabschnitten zu einem äußeren Schlauchabschnitt umgestülpt ist. der sich hierdurch ausbildende geschlossene Ringraum ist mit einem Schmiermittel angefüllt. Wie ferner aus der WO 88/01924 zu erkennen ist, kann der Stülpschlauch mit einer zusätzlichen Armierung (interior layer 32A, outermost) versehen sein. Dabei ist der äußere Schlauchabschnitt dadurch geschlossen, indem die beiden aufeinander zulaufenden freien Enden des Stülpschlauchs sich übereinander überlappen und verklebt sind. Als eine zusätzliche Maßnahme zur Sicherung der Klebestelle wird die Anordnung eines separaten Segments aus einem flexiblen Material angegeben wie bspw. ein Klebestreifen und dergleichen.

Angesichts dieses Stands der Technik besteht die Aufgabe der vorliegenden Erfindung darin, eine Stülpschlauchkonstruktion bereitzustellen, mittels dem eine erhöhte Vorschubkraft auf einen Endoskopschaft, einen Katheter oder ein ähnliches schaftartiges Rundmaterial übertragen werden kann.

Diese Aufgabe wird erfindungsgemäß mittels einer Stülpschlauchkonstruktion mit den Merkmalen gemäss dem Anspruch 1 gelöst.

Der Erfindungsgegenstand gemäß dem geltenden Patentanspruch 1 sieht zur Lösung dieser Aufgabe die Anordnung einer Schlauchführungshülse vor, wobei der innere Schlauchabschnitt durch diese Schlauchführungshülse gleitend geführt ist, wohingegen die aufeinander zulaufenden freien Enden des Stülpschlauchs an den beiden axialen Endabschnitten der Schlauchführungshülse fixiert sind. Hierdurch bildet sich zwischen den beiden aufeinander zulaufenden Enden des Stülpschlauchs ein exponierter Abschnitt der Schlauchführungshülse, welcher als Anschlussfläche für einen Schlauchantrieb genutzt werden kann. Durch die Schlauchführungshülse wird ein Aufschuppen des Stülpschlauchs vermindert, sodass eine höhere Vorschubkraft auf ein hierdurch anzutreibendes Gerät aufbringbar ist.

Im übrigen sei noch auf die US 5,171,305 als ein weiterer Stand der Technik verwiesen, aus der ebenfalls ein einfach gestülpter Schlauch für das Antreiben eines Trokars bekannt ist.

Vorteilhaft ist es hierbei, wenn die Armierung aus einer Wicklung besteht, die durch einen Faden oder Fadengespinnst vorzugsweise aus Nylon gebildet ist. Wichtig hierbei ist, dass die Armierung eine geringfügige elastische Aufweitung des Schlauchs insbesondere zu Montagezwecken zulässt, welche durch das verwendete Nylonmaterial infolge von dessen Eigenelastizität ermöglicht wird. Alternativ kann natürlich auch ein Material mit geringerer Eigenelastizität wie beispielsweise ein Draht aus Metall verwendet werden. In diesem Fall ist der Draht jedoch nicht gerade sondern schlangen- oder Zick-Zack förmig gezogen, um eine bestimmte Dehnung und damit ein Aufweiten des Schlauchs zuzulassen.

Weitere vorteilhafte Ausgestaltungen der Erfindung sind dabei Gegenstand der Unteransprüche.

Die Erfindung wird nachstehend anhand eines bevorzugten Ausführungsbeispiels unter Bezugnahme auf die begleitenden Zeichnungen näher erläutert.
Die Fig. 1 zeigt einen Längsschnitt einer Stülpschlauchkonstruktion gemäss einem bevorzugten Ausführungsbeispiel der vorliegenden Erfindung für die Verwendung insbesondere bei einem Endoskopiegerät oder einem Katheter,
Fig. 2 zeigt eine Perspektivenansicht eines äußeren Endabschnitts der Stülpschlauchkonstruktion gemäss dem bevorzugten Ausführungsbeispiel und
Fig. 3 zeigt einen Querschnitt der erfindungsgemäßen Stülpschlauchkonstruktion.

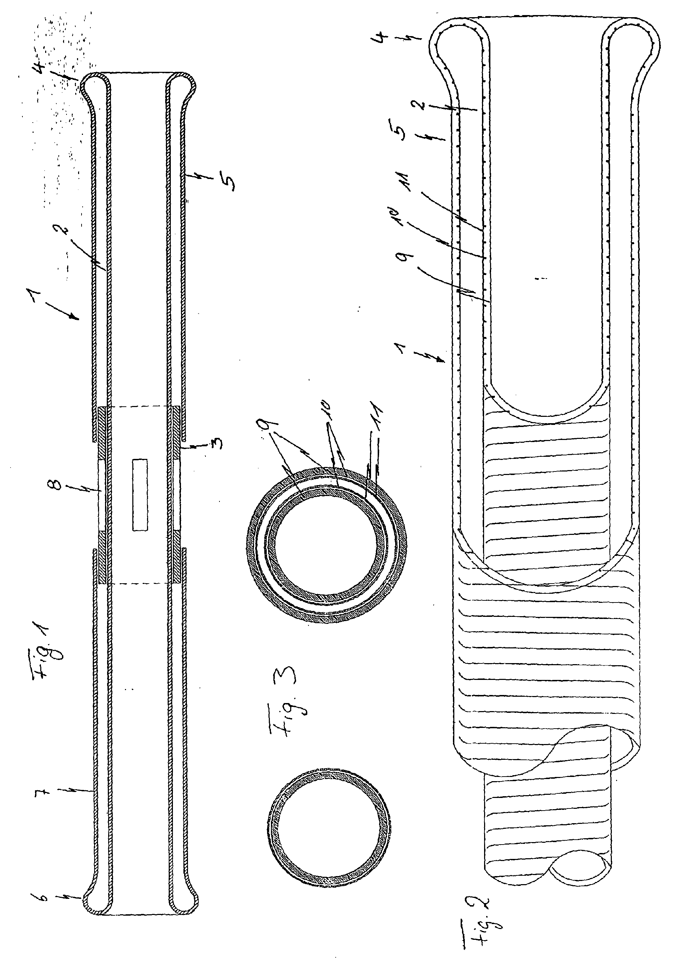

Wie aus der Fig. 1 zu entnehmen ist, umfasst die erfindungsgemäße Stülpschlauchkonstruktion einen Stülpschlauch 1 bestehend aus einem inneren Schlauchabschnitt 2, der durch eine Antriebs- und Führungshülse bzw. ein Schlauchführungsteil 3 gleitfähig mit einem dazwischen sich ausbildenden Ringspalt hindurchgeführt und in seinem vorderen Bereich (Umstülpbereich) 4 zu einem vorderen, äußeren Schlauchabschnitt 5 umgestülpt ist. Der vordere, äußere Schlauchabschnitt 5 ist dabei zu der Antriebs- und Führungshülse (Schlauchführungsteil) 3, welche aus einem steifen Material, vorzugsweise einem Kunststoffmaterial oder einem Metall besteht, zurückgeführt und an einem axialen Hülsenendabschnitt an der Antriebshülse 3 derart befestigt, dass diese zwischen dem inneren Schlauchabschnitt 2 und dem vorderen, äußeren Schlauchabschnitt 5 zu liegen kommt. D.h. mit anderen Worten, das Ende des vorderen, äußeren Schlauchabschnitts 5 ist auf der äußeren Mantelfläche der Antriebshülse 3 in deren einem axialen Endbereich vorzugsweise angeklebt oder anvulkanisiert. Alternativ kann auch eine andersartige Fixierung, wie beispielsweise eine Schlauchklemme und dergleichen vorgesehen sein.

In einem hinteren Bereich (Umstülpbereich) 6 des Stülpschlauchs 1 ist der innere Schlauchabschnitt 2 zu einem hinteren, äußeren Schlauchabschnitt 7 umgestülpt, der ebenfalls zu der Antriebshülse 3 zurückgeführt und an einem axialen Ende der Antriebshülse 3 in gleicher Weise, wie vorstehend beschrieben fixiert ist. Dabei kommt dieses axiale Ende der Antriebshülse 3 ebenfalls zwischen dem inneren und äußeren, hinteren Schlauchabschnitt 2 und 7 zu liegen. Die Antriebshülse 3 dient zum einen als ein Führungselement für den inneren Schlauchabschnitt 2, um Aufwerfungen sowie Falten- bzw. Schuppenbildung zu verhindern und zum anderen als ein Verbindungsstück des vorderen, äußeren Schlauchabschnitts und des hinteren, äußeren Schlauchabschnitts 7, wobei ein Mittenbereich der Antriebshülse 3 an dessen äußerer Mantelfläche exponiert, d. h. nicht von dem Stülpschlauch 1 überdeckt verbleibt. In diesem Mittenabschnitt weist die Antriebshülse 3 zumindest eine Öffnung 8, vorzugsweise einen in Axialrichtung sich erstreckenden Längsschlitz vor bestimmter Breite auf. Vorliegend sind vier oder mehr, im gleichen Winkelabstand zueinander angeordnete Längsschlitze 8 vorgesehen, von denen zwei diametrisch gegenüberliegende Längsschlitze in der Fig. 1 gezeigt werden. Des weiteren hat die Antriebshülse 3 vorzugsweise an ihrer Innenseite eine Anzahl von nicht näher dargestellten durchgehenden Längsnuten, die sich an den Stirnseiten der Antriebshülse 3 in Hohlräume öffnen, die sich zwischen dem inneren Schlauchabschnitt 2 und den äußeren Schlauchabschnitten 5, 7 ausbilden. Diese Längsnuten können dabei entweder achsparallel oder spiralförmig gezogen sein.

Wie insbesondere in der Fig. 1 zu erkennen ist, ist das Material, d. h. die Materialart sowie die Materialstärke des Stülpschlauchs 1 derart gewählt, dass sich im vorderen und hinteren Stülpbereich 4, 6 jeweils eine wulstförmige Aufweitung infolge einer Materialanhäufung an der Umstülpung ausbildet.

Die Materialart sowie die Materialstärke des bevorzugten Ausführungsbeispiels der Erfindung wird nachfolgend anhand der Fig. 2 und 3 im einzelnen beschrieben.

Der Stülpschlauch 1 gemäss dem bevorzugten Ausführungsbeispiel besteht aus einem zu einem Schlauch 9 extrudierten Silikonmaterial mit einer Wandstärke von 0,5 bis 1,5 mm vorzugsweise 0,8 mm. Dieser Silikonschlauch ist von einem Armierungsmaterial 10 vorzugsweise aus Nylon umgeben (nachfolgend als Nylonwickel oder auch Drahtwickel bezeichnet), welches wiederum von einer Hülle 11 aus Silikon überzogen ist. Die Wandstärke der Hülle 11 beträgt dabei 0,1 bis 0,5 mm vorzugsweise 0,2 mm. Bei dem Versteifungsmaterial 10 aus Nylon handelt es sich, wie insbesondere aus der Fig. 2 zu entnehmen ist, um einen Nylonfaden bzw. zu einem fadengesponnenen Nylongespinnst, der in Axialrichtung des Silikonschlauchs 1 um dessen Mantelfläche mit einer Steigung von 0,2 bis 2 mm vorzugsweise 0,5 mm unter einer vorbestimmten Zugspannung aufgewickelt ist. Die Hülle 11 aus Silikon füllt dabei die Spalte (Abstände) zwischen den einzelnen Nylonfäden der Armierung bzw. des Nylonwickels 10 aus und deckt dabei die Armierung 10 vollständig nach außen ab. Die vorstehenden Angaben bezüglich der Dimensionierung des Stülpschlauchs und des Nylonwickels beziehen sich auf eine Stülpschlauchkonstruktion insbesondere für medizinische Zwecke, beispielsweise für einen Endoskopschaft oder einen Katheter. Diese kann jedoch abweichend sein, insbesondere dann, wenn die Stülpschlauchkonstruktion zur Untersuchung und Bearbeitung von Rohren oder Schächten wird, die auf andere Weise nicht oder nur schwer zugänglich sind. Auch ist das Material Nylon nur als ein bevorzugtes Ausführungsbeispiel angegeben und kann ohne weiteres durch ein anderes Material mit ähnlichen Eigenschaften ersetzt werden. Auch können die Eigenschaften von Nylon durch konstruktive Maßnahmen simuliert werden wie nachfolgend kurz dargestellt wird.

Wie eingangs bereits angedeutet wurde, muss die Armierung 10 eine geringe Aufweitung des Schlauchs zulassen, damit dieser montiert werden kann. Das verwendete Nylogespinnst erlaubt eine solche Aufweitung infolge der Eigenelastizität. Für den Fall, dass z.B. ein Drahtwickel verwendet wird, muss der Draht beispielsweise in Zick-Zack-Form gezogen sein, um in Drahtlängsrichtung geringfügig elastisch gestreckt werden zu können.

Die Antriebshülse 3 dient dabei zur Befestigung des nicht weiter dargestellten Antriebsmechanismusses, dessen Gehäuse auf die Antriebshülse 3 aufgeklemmt wird, derart, dass die sich gegenüberliegenden, an der Antriebshülse 3 festgeklebten Enden des Stülpschlauchs 1 zwischen der Antriebshülse 3 und dem Antriebsmechanismus bzw. dessen Gehäuse quasi als Dichtungen zu liegen kommen. Dieser Antriebsmechanismus hat ebenfalls nicht gezeigte Reibräder, die bei dessen Befestigung auf der Antriebshülse 3 durch die in der Fig. 1 dargestellten Schlitze 8 ragen. In den Stülpschlauch 1 wird schließlich der ebenfalls nicht gezeigte Endoskopschaft oder ein schaftähnliches Rundmaterial wie zum Beispiel ein Katheter eingeführt, dessen Durchmesser geringfügig kleiner als der Innendurchmesser des inneren Schlauchabschnitts 2 des Stülpschlauches 1 ist, wobei die Reibräder über den inneren Schlauchabschnitt 2 gegen den nicht dargestellten Endoskopschaft drücken.

Versuche der Erfinderin anhand eines Endoskopiegeräts haben ergeben, dass der Stülpschlauch 1 mit dem vorstehend beschriebenen Nylonwickel 10 zusätzlich zu der Führungshülse 3, bzw. der vorstehend beschriebene konstruktive Aufbau des Stülpschlauchs 1 gegenüber einem nicht ausgesteiften, ausschließlich aus Silikon oder einem anderen Material (z.B Neopren) bestehenden Stülpschlauch eine um den Faktor 3 vergrößerte Schubkraft in Axialrichtung auf den Endoskopschaft übertragen kann, bevor er die Tendenz zur Schuppenbildung zeigt. Dabei wird durch die vorstehend beschriebenen Nylonwickel 10 das Stülpverhalten nur geringfügig beeinflusst, so dass ein Wirkungsverlust d.h. ein Ansteigen der notwendigen Antriebskraft durch den Umstülpvorgang während des Eindringens des Endoskops minimal bleibt.

Abschließend sei darauf hingewiesen, dass der konstruktive Aufbau des Stülpschlauchs 1 insbesondere die Ausbildung der Armierung 10 gemäss der Erfindung nicht auf den Stülpschlauch der Doppelstülpbauart beschränkt ist. Die Stülpschlauchkonstruktion lässt sich auch bei einfach gestülpten Stülpschläuchen vorteilhaft anwenden, bei denen der hintere Umstülpbereich, der gemäss vorstehender Beschreibung eine Bremskraft entgegen der Vorschubkraft der Reibräder für ein Synchronisieren der Vorschubgeschwindigkeiten des Endoskopschaftes und des Stülpschlauchs aufnimmt, durch eine weitere Antriebs- bzw.

Bremseinrichtung ersetzt ist, die unabhängig zu der eigentlichen Antriebseinrichtung ausschließlich auf den Endoskopschaft einwirkt und daher lediglich nur eine Umstülpung im vorderen Bereich des Stülpschlauchs für die Vorschubbewegung des Endoskopschafts und des Stülpschlauchs erforderlich ist.

Des weiteren ist die Erfindung nicht auf die ausschließliche Verwendung von Silikon als Schlauchmaterial beschränkt, sondern es können auch andere Materialien mit gleichen oder ähnlichen Eigenschaften bezüglich Verarbeitung, Fliess- und Elastizitätsverhalten, Verträglichkeit für den menschlichen Körper, Reaktionsverhalten mit Schmiermitteln usw. als Basismaterial verwendet werden.

## Patentansprüche

1. Stülpschlauchkonstruktion als Transportmittel vorzugsweise für einen flexiblen Endoskopschaft, einen als flexiblen Schaft ausgebildeten Katheter oder Operationstubus und dergleichen Schaftmaterial mit einem Stülpschlauch (1) bestehend aus einem inneren Schlauchabschnitt (2), der an zumindest einem Umstülpbereich (4) zu einem äußeren Schlauchabschnitt (5) umgestülpt ist, wobei
der Stülpschlauch (1) mit einer Armierung (10) versehen ist, **gekennzeichnet durch** eine Schlauchführungshülse (3), **durch** die der innere Schlauchabschnitt (2) läuft und an deren axialen Endabschnitten die Enden des vorderen, äußeren Schlauchabschnitts (5) sowie eines hinteren, äußeren Schlauchabschnitts (7) fixiert sind, welcher **durch** Umstülpen des inneren Schlauchabschnitts (2) an einem hinteren Umstülpbereich (6) gebildet ist.

2. Stülpschlauchkonstruktion nach Anspruch 1, **dadurch gekennzeichnet, dass** die Armierung (10) aus einer Wicklung besteht.

3. Stülpschlauchkonstruktion nach Anspruch 2, **dadurch gekennzeichnet, dass** die Wicklung durch einen Faden oder Fadengespinnst vorzugsweise aus Nylon gebildet ist.

4. Stülpschlauchkonstruktion nach Anspruch 2 oder 3, **dadurch gekennzeichnet, dass** die Wicklung eine Steigung von 0, 2 bis 2 mm hat.

5. Stülpschlauchkonstruktion nach Anspruch 2 oder 3, **dadurch gekennzeichnet, dass** die Wicklung eine Steigung von 0,5 mm hat.

6. Stülpschlauchkonstruktion nach einem der vorstehenden Ansprüche, **dadurch gekennzeichnet, dass** der Stülpschlauch (1) aus einem inneren Schlauch (9) vorzugsweise aus Silikon besteht, auf den die Armierung (10) aufgezogen ist, die wiederum mit einer Hülle (11) vorzugsweise aus Silikon umgeben ist.

7. Stülpschlauchkonstruktion nach Anspruch 6, **dadurch gekennzeichnet, dass** die Wandstärke des inneren Schlauchs (9) 0,5 bis 2,5 mm und vorzugsweise 0,8 mm beträgt.

8. Stülpschlauchkonstruktion nach Anspruch 6 oder 7, **dadurch gekennzeichnet, dass** die Wandstärke der Hülle 0,1 bis 0,5 mm und vorzugsweise 0,2 mm beträgt.

9. Stülpschlauchkonstruktion nach Anspruch 2, **dadurch gekennzeichnet, dass** die Wicklung aus einem Draht vorzugsweise aus Metall besteht.

10. Stülpschlauchkonstruktion nach Anspruch 9, **dadurch gekennzeichnet, dass** der Draht Zick-Zack förmig oder schlangenförmig gezogen ist.

11. Stülpschlauchkonstruktion nach einem der vorstehenden Ansprüche, **dadurch gekennzeichnet, dass** die Armierung (10) eine geringfügige Aufweitung des Stülpschlauchs (1) zulässt.

12. Stülpschlauchkonstruktion nach einem der vorstehenden Ansprüche 2 bis 11, **dadurch gekennzeichnet, dass** die Steigung der Wicklung in Abhängigkeit von der Stülpschlauchlänge in Schlauchlängsrichtung unterschiedlich ist.

## Claims

1. An inverted sleeve structure as a transport means preferably for a flexible endoscope shaft, a catheter or operating tube constructed as a flexible shaft and similar shaft devices, having an inverted sleeve (1) consisting of an inner sleeve portion (2), which is folded back at at least one fold-back area (4) to form an outer sleeve portion (5),
the inverted sleeve (1) being provided with a reinforcement (10), **characterised by** a sleeve guiding bush (3), through which the inner sleeve portion (2) extends and to the axial end portions of which the ends of the front, outer sleeve portion (5) and of a rear, outer sleeve portion (7) are fixed, the latter being formed by folding back the inner sleeve portion (2) at a rear fold-back area (6).

2. An inverted sleeve structure according to claim 1, **characterised in that** the reinforcement (10) consists of a winding.

3. An inverted sleeve structure according to claim 2, **characterised in that** the winding is formed by a filament or spun filament, preferably of nylon.

4. An inverted sleeve structure according to claim 2 or claim 3, **characterised in that** the winding has a pitch of 0.2 to 2 mm.

5. An inverted sleeve structure according to claim 2 or claim 3, **characterised in that** the winding has a pitch of 0.5 mm.

6. An inverted sleeve structure according to one of the preceding claims, **characterised in that** the inverted sleeve (1) consists of an inner sleeve (9) preferably of silicone, on which the reinforcement (10) is mounted, which is in turn surrounded by a cover (11) preferably of silicone.

7. An inverted sleeve structure according to claim 6, **characterised in that** the wall thickness of the inner sleeve (9) amounts to from 0.5 to 2.5 mm and preferably to 0.8 mm.

8. An inverted sleeve structure according to claim 6 or claim 7, **characterised in that** the wall thickness of the cover amounts to from 0.1 to 0.5 mm and preferably to 0.2 mm.

9. An inverted sleeve structure according to claim 2, **characterised in that** the winding consists of a wire, preferably of metal.

10. An inverted sleeve structure according to claim 9, **characterised in that** the wire is laid in zigzag or coil formation.

11. An inverted sleeve structure according to one of the preceding claims, **characterised in that** the reinforcement (10) permits slight expansion of the inverted sleeve (1).

12. An inverted sleeve structure according to one of preceding claims 2 to 11, **characterised in that** the pitch of the winding differs as a function of the inverted sleeve length in the longitudinal direction of the inverted sleeve.

## Revendications

1. Construction à tuyau retroussé faisant office de moyen de transport, de préférence pour une tige d'endoscope flexible, un cathéter réalisé sous forme de tige flexible, ou un tube d'opération ou un matériau en tige analogue, avec un tuyau retroussé (1), composé d'un tronçon de tuyau (2) intérieur, retroussé en au moins une zone de retroussement (4) pour former un tronçon de tuyau extérieur (5), où
le tuyau retroussé (1) est muni d'une armature (10), **caractérisée par** une douille de guidage de tuyau (3), à travers laquelle passe le tronçon de tuyau intérieur (2) et sur les tronçons d'extrémité axiaux de laquelle sont fixées les extrémités du tronçon de tuyau extérieur (5) avant, ainsi que d'un tronçon de tuyau extérieur (7) arrière, formé par un retroussement du tronçon de tuyau intérieur (2) sur une zone de retroussement arrière (6).

2. Construction à tuyau retroussé selon la revendication 1, **caractérisée en ce que** l'armature (10) est formée d'un enroulement.

3. Construction à tuyau retroussé selon la revendication 2, **caractérisée en ce que** l'enroulement est formé par un fil ou est filé en un fil, de préférence en nylon.

4. Construction à tuyau retroussé selon la revendication 2 ou 3, **caractérisée en ce que** l'enroulement présente un pas de 0,2 à 2 mm.

5. Construction à tuyau retroussé selon la revendication 2 ou 3, **caractérisée en ce que** l'enroulement présente un pas de 0,5 mm.

6. Construction à tuyau retroussé selon l'une des revendications précédentes, **caractérisée en ce que** le tuyau retroussé (1) est formé d'un tuyau intérieur (9), de préférence en silicone, sur lequel est enfilée l'armature (10), qui, à son tour, est entourée par une gaine (11), de préférence en silicone.

7. Construction à tuyau retroussé selon la revendication 6, **caractérisée en ce que** l'épaisseur de paroi du tuyau intérieur (9) est de 0,5 à 2,5 mm, de préférence de 0,8 mm.

8. Construction à tuyau retroussé selon la revendication 6 ou 7, **caractérisée en ce que** l'épaisseur de paroi de la gaine est de 0,1 à 0,5 mm, et de préférence de 0,2 mm.

9. Construction à tuyau retroussé selon la revendication 2, **caractérisée en ce que** l'enroulement est formé d'un fil, de préférence en métal.

10. Construction à tuyau retroussé selon la revendication 9, **caractérisée en ce que** le fil est formé en zigzag, ou est étiré en serpentin.

11. Construction à tuyau retroussé selon l'une quelconque des revendications précédentes, **caractérisée en ce que** l'armature (10) permet un léger évasement du tuyau retroussé (1).

12. Construction à tuyau retroussé selon l'une des revendications 2 à 11 précédentes, **caractérisée en ce que** le pas de l'enroulement est différent en fonction de la longueur de tuyau retroussé, observée dans la direction longitudinale du tuyau.
